# EUROPEAN PATENT SPECIFICATION

(11) **EP 3 512 889 B1**
(45) Date of publication and mention of the grant of the patent: **31.12.2025**
(21) Application number: 16916367.2
(22) Date of filing: 16.09.2016
(51) Int. Cl.: C08F 10/06, C08F 2/01, C07C 5/42, C07C 7/09, C07C 9/08, C07C 11/06, C08F 2/34, C08J 11/00, B01J 19/00, B01J 8/00

(54) **INTEGRATED PROPANE DEHYDROGENATION PROCESS**
INTEGRIERTES PROPANDEHYDRIERUNGSVERFAHREN
PROCÉDÉ INTÉGRÉ DE DÉSHYDROGÉNATION DU PROPANE

(43) Date of publication of application: 24.07.2019
(73) Proprietor: Lummus Technology LLC, Bloomfield NJ 07003-3096 (US)
(72) Inventor: PANDITRAO, Sunil, Shashikant, Hackettstown NJ 07840 (US); CARRILLO, Alejandro, Salvador, New York NY 10024 (US)
(74) Representative: Osha BWB
(86) International application number: PCT/US2016/052081
(87) International publication number: WO 2018/052437

(56) References cited:
- WO-A1-2005/030811
- US-A- 5 457 256
- US-A1- 2007 249 793
- US-A1- 2010 025 218
- US-A1- 2010 331 589
- US-A1- 2013 158 327
- US-A1- 2015 158 795

## Description

### BACKGROUND

Production of propylene via dehydrogenation of propane conventionally involves a dehydrogenation reactor and a separation system to recover a propylene product stream, such as a polymer grade propylene (98wt%+) stream. The propylene produced may then be used in any number of downstream processes, one of which includes converting the propylene to a polymer.

Production of polypropylene, for example may be performed by many various processes, including gas phase polymerization processes and bulk fluid polymerization processes. These processes conventionally use a gas, such as nitrogen, during the polymerization process, for example as a fluidization gas or purge gas, or to remove light hydrocarbons from the polymerization product, where the nitrogen and other entrained gases may be collected as a purge gas. In some polypropylene processes, a gas membrane separation unit may be used to separate purge gas (nitrogen) from light hydrocarbons. The light hydrocarbons are typically flared while the nitrogen may be recycled. The operation of the gas membrane separation unit requires a compressor, which is very energy intensive, partially due to the general power requirements to compress light gasses, and partially due to the fact that it has to be sized to accommodate situations ranging from no flow all the way to full output. Gas phase polymerization processes also require a carrier, for which a separate, expensive recovery process is required.

US 2007/249793 A1 describes a simplified process for preparing polyolefins from saturated hydrocarbons. The process involves partial and selective dehydrogenation of a saturated hydrocarbon in the presence of oxygen to form an olefin, unreacted hydrocarbon, and water, and optionally other by-products (such as carbon dioxide and/or partial oxidation products) and oxygen. The water, other by-products (if present), and oxygen (if present) are separated from the olefin and unreacted hydrocarbon. No other separation is performed. The olefin and unreacted hydrocarbon are polymerized in the presence a polymerization catalyst or initiator to make polyolefin. Solid polyolefin is separated from unreacted hydrocarbon, which is recycled to the dehydrogenation reaction.

WO 2005/030811 A1 describes a process for the polymerization of propylene from a feed comprising propane and propylene. A C3-splitter is used, fed from a propane/propylene mixture generated in a propane dehydrogenation unit. The propane may be recycled to the dehydrogenation unit, and the de-propanized propylene is fed to the polymerization reactor.

### SUMMARY OF THE CLAIMED EMBODIMENTS

Embodiments disclosed herein integrate propylene production with propylene polymerization, reducing the capital requirements and operating expenses as compared to operating both processes as stand-alone units.

In one aspect, embodiments disclosed herein relate to a process for the production of polypropylene where a hydrocarbon feedstock, containing propane, is fed to a propane dehydrogenation reaction zone to convert a portion of the propane to propylene. The propane and propylene are separated, and at least a portion of the propylene stream is fed to a polymerization zone. In the polymerization zone, propylene is reacted to produce a polymerization product, and also recovered is a purge gas and a carrier gas stream. The purge and carrier gases are independently fed to the separation system. The purge gas comprises a gas containing nitrogen and optionally one or more of propane, propylene, ethylene, or hydrogen. The carrier gas comprises one or more of propane, propylene, ethylene, hydrogen, or nitrogen. When the carrier gas comprises nitrogen, the carrier gas is leaner in nitrogen relative to the purge gas. The separating comprises compressing the dehydrogenated effluent in a product gas compressor, cooling the compressed effluent in a cold box, and separating the cooled, compressed effluent in a deethanizer and a C3 splitter. The process further comprises independently passing the purge gas and carrier gas through the cold box, thereby cooling the purge gas and carrier gas; and condensing, in the cold box, a portion of the carrier gas and a portion of the purge gas. The process further comprises recovering a carrier gas condensate stream and a carrier gas vapor stream; feeding the carrier gas condensate stream to the C3 splitter and feeding the carrier gas vapor stream to the product gas compressor. The process further comprises recovering a purge gas condensate stream and a purge gas vapor stream and vaporizing the purge gas condensate stream in the cold box; feeding the vaporized purge gas condensate stream to the product gas compressor and feeding the purge gas vapor stream to the polymerization zone.

In another aspect, embodiments disclosed herein relate to a system for the integrated production of propylene and polypropylene. The system contains a propane dehydrogenation reaction zone configured to convert at least a portion of a hydrocarbon feedstock comprising propane to propylene, forming a dehydrogenated effluent; a separation system configured to separate the dehydrogenated effluent and form a propane stream and a propylene stream; a polymerization zone configured to convert at least a portion of the propylene stream to polypropylene, and produce a polymerization product, a carrier gas stream, and a purge gas stream; a flow line for feeding the carrier gas stream to the separation system; and a flow line for feeding the purge gas stream to the separation system. The purge gas comprises a gas containing nitrogen and optionally one or more of propane, propylene, ethylene, or hydrogen, and the carrier gas comprises one or more of propane, propylene, ethylene, hydrogen, or nitrogen. When the carrier gas comprises nitrogen, the carrier gas is leaner in nitrogen relative to the purge gas. The separation system comprises a product gas compressor for compressing the dehydrogenated effluent, a cold box for cooling the compressed effluent, a deethanizer configured to separate the cooled, compressed effluent to recover a C2- stream and a C3+ stream, and a C3 splitter configured to produce an overhead product comprising propylene and a bottoms product comprising propane. The cold box comprises a pass for cooling the purge gas stream; a separator, within or exterior to the cold box, for receiving the cooled purge gas stream and recovering a purge gas condensate stream and a purge gas vapor stream; a pass for vaporizing the purge gas condensate stream; and a pass for cooling the carrier gas stream, producing a carrier gas condensate stream and a carrier gas vapor stream. The system further comprises a line for feeding the vaporized purge gas condensate stream to the product gas compressor; a line for feeding the purge gas vapor stream to the polymerization zone; a line for feeding the carrier gas condensate stream to the C3 splitter; and a line for feeding the carrier gas vapor stream to the product gas compressor.

Other aspects and advantages will be apparent from the following description and the appended claims, which define embodiments of the process and the system.

### BRIEF DESCRIPTION OF DRAWINGS

Figure 1 illustrates a simplified block diagram of a propane dehydrogenation process that may be useful with embodiments disclosed herein.
Figure 2 illustrates a simplified process flow diagram of a propylene polymerization process that may be useful with embodiments disclosed herein.
Figure 3 illustrates a prior art method for separating various off-gases from a polymerization process.
Figure 4 is a simplified block diagram of an integrated process for producing propylene and polypropylene according to embodiments herein.
Figure 5 illustrates one approach to integrate propane dehydrogenation and propylene polymerization processes (not forming part of the claimed subject-matter).
Figure 6 illustrates an integrated propane dehydrogenation and propylene polymerization processes according to embodiments herein.

### DETAILED DESCRIPTION

In one aspect, embodiments herein relate to a process for producing polypropylene from a propane feedstock. The process utilizes non-conventional product recycle and heat integration between a polymerization reaction zone and an upstream dehydrogenation reaction zone.

A hydrocarbon feedstock comprising propane is fed to a propane dehydrogenation reaction zone. Within the dehydrogenation zone, the hydrocarbon feedstock may be contacted with a catalyst at appropriate reaction conditions to convert a portion of the propane to propylene. A reaction effluent, including propane and propylene, is then recovered from the dehydrogenation reaction zone. The reaction effluent is then separated in a separation system to recover at least two separate fractions, a high purity or polymer grade propylene stream and a stream including unreacted propane. The high purity propylene stream is then fed to a polymerization reaction zone, where the propylene is reacted to produce a polymer product which may be separated from residual light gases in a polymerization effluent separation zone.

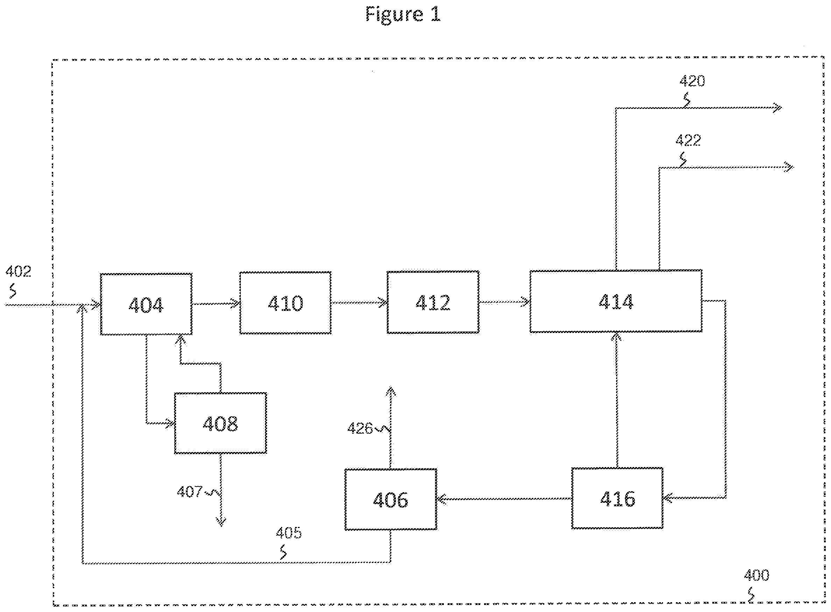

A simplified process flow diagram of a propane dehydrogenation process is illustrated in Figure 1. A propane dehydrogenation unit 400 may include a vaporizer 404 to vaporize a propane-containing feedstock 402 and recycle propane / heavies 405 recovered from a C3 (propane / propylene) splitter 406. A deoiler (or depropanizer) 408 may be used to remove the heavies 407 not vaporized. A portion of the propane is then converted to propylene in reactor 410, the effluent then being compressed in product gas compressor 412, cooled in cold box / exchanger 414, and separated using a deethanizer (C2/C3 splitter) 416 and the C3 splitter 406. Products recovered from propane dehydrogenation unit 400 may include, among others, hydrogen 420, light hydrocarbons 422, and propylene product 426. The propylene product 426 recovered, or a portion thereof, is then converted to polypropylene or a propylene polymer or copolymer in a polymerization zone.

The polymerization zone may include any conventional type of reactor, including fluid bed reactors, gas phase reactors, slurry reactors, or linear flow reactors. Likewise, the polymerization process may be catalyst by any number of catalysts, including metallocene, Ziegler-Natta, and various chromium based catalysts, among others. Further, the polymerization zone may include feed of co-monomers and chain termination agents, such as ethylene and hydrogen, respectively, among many other feeds known to be used in the art.

Nitrogen may be used to control head space compositions or may be used as a fluidization gas. Further, polypropylene product that leaves the polypropylene reactor may contain various components, including unreacted propylene, propane, unreacted comonomers, hydrogen, as well as incompletely formed polymers, solvents, and other components used in the process. These components may be removed from the polypropylene by way of a purge stream, which includes nitrogen, using a polymer separation system. The polymer separation system may include drying, centrifuge, cyclone separators, flashing, cooling, distillation, absorption, or combinations of these, and the particular separation system used may depend on the type of polymerization reactor used as well as the other feed components used.

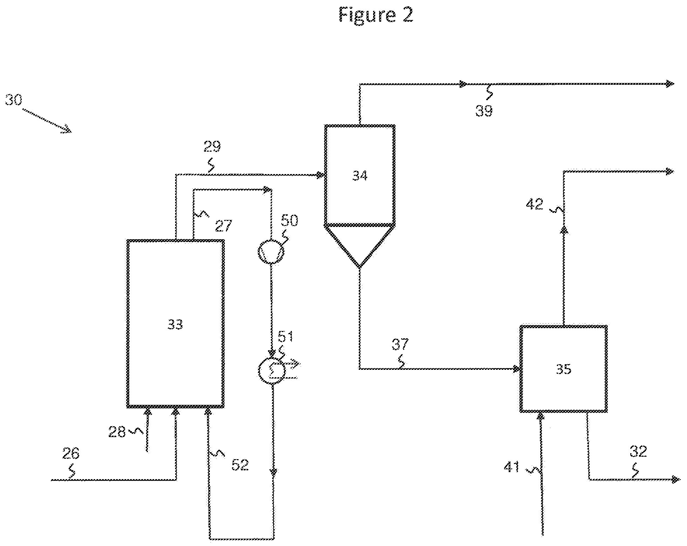

Referring now to Figure 2, a polymerization reaction zone 30 is illustrated. While polymerization zone components may differ, depending upon the polymerization reactor type, components fed, and separation systems used, the integration of a propylene polymerization system and a propane dehydrogenation system according to embodiments herein is exemplified. One skilled in the art may readily expand the teachings associated with Figure 2 to other propylene polymerization systems.

Propylene stream 26 is fed to a polymerization reactor 33, which may be a fluidized bed gas phase reactor, for example. Recycle stream 52, which may be liquid propylene, and comonomer stream 28, which may also contain recycled propylene, may also be fed to polymerization reactor 33. The production of the propylene polymers may be carried out in any gas phase reactor suitable for the polymerization of alk-1-enes, either batch-wise, semi-continuously, or continuously, such as in fluidized bed reactors or horizontal or vertical stirred powder bed reactors. It will be understood that the polymerization may be carried out in a series of consecutively coupled reactors. The reaction time depends on the chosen reaction conditions. In general, the reaction time is from about 0.2 to about 20 hours, usually from about 0.5 to about 10 hours.

Unreacted propylene may be recovered via stream 27 and fed to compressor 50 and condenser 51, forming recycle stream 52. Recycle stream 52 may provide cooling to polymerization reactor 33. The polymerization reaction is exothermic and reactor cooling may be achieved by flash evaporation of recycle stream 52 in polymerization reactor 33, where recycled stream 52 is mixed with propylene stream 26, and / or comonomer stream 28 in the polymerization reactor 33, and evaporated.

In general, the polymerization is carried out at a temperature in the range of from about 20°C to about 150°C, such as from about 50°C to about 120°C, or from about 60°C to about 90°C, and a pressure in the range of from about 1 to 100 bar, such as from about 15 to about 60 bar, or from about 20 to about 45 bar.

Polymerization effluent 29, containing polypropylene and carrier gas, among other unreacted feed components, and byproducts, may be recovered from the polymerization reactor. Polymerization product 29 may then be fed to a separation unit 34, such as a cyclone separator. The separation unit 34 separates polypropylene product 37 from carrier gas 39, which may contain unreacted monomer. Polypropylene product 37 may then be fed to a purge gas unit 35 along with purge gas 41, which may include nitrogen. The purge gas unit 35 may separate residual gas contained in the polypropylene product, and recovered as a purge gas stream 42. Degassed polypropylene product may be recovered via stream 32. Carrier gas 39 and purge gas stream 42 may be fed to a downstream separation system (not shown), integrating C3 production and polymerization according to embodiments disclosed herein.

While polymerization reaction systems and polymer product recovery systems may vary, propylene polymerization processes typically result in one or more off gas streams, including carrier gas 39 and purge gas stream 42, and a polymer product 32. As a result of the fluidization or head space control, as well as polymer product purifications, the polymerization reaction zone may produce an off-gas, including propane and nitrogen, among other valuable components, which may be separated and recovered for re-use.

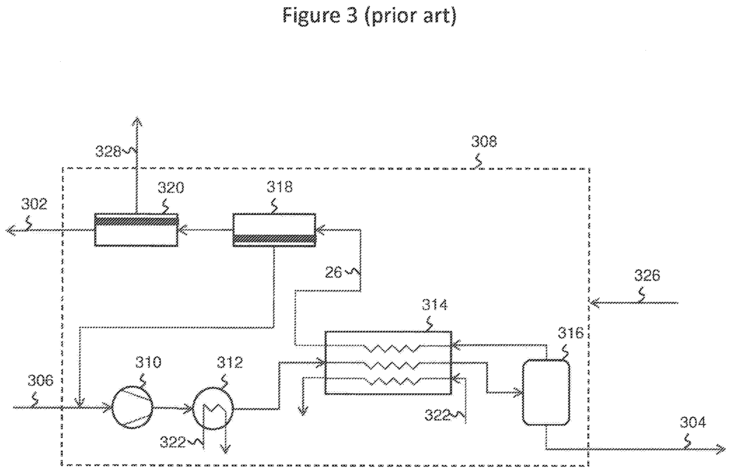

In some processes, such as that described in US7067597, propylene is recovered and recycled, however the remaining components are discarded. The propane, or other components present, may be flared, for example. As another example, a system such as that illustrated in Figure 3 may be used, where, in the polypropylene unit, nitrogen 302 and hydrocarbons 304 are separated from the purge gas 306 in a membrane unit 308, which may include a compressor 310, aftercooler 312, condenser 314, gas/liquid separator 316, one or more membranes 318, 320, and one or more refrigerant streams 322, and enclosure 308 atmosphere control streams 326. In the example process of Figure 3, stream 328, which may include non-condensables or uncondensed heavier components, is forwarded to a flare, while nitrogen 302 and hydrocarbons 304 may be recovered for reuse.

These and other streams produced as products or byproducts of current propylene polymerization processes are generally processed in inefficient manners, or flared, resulting in excess operating costs, poor energy usage, and higher emissions to the atmosphere. In contrast, embodiments disclosed herein effectively and efficiently integrate a propane dehydrogenation reaction zone, dehydrogenation effluent separations, a polymerization reaction zone, and polymerization effluent separations, as will be described further below.

In various conventional designs for propane dehydrogenation plants, the propane/propylene splitter column is a low pressure splitter that is driven by a heat pump and heat exchanger. The heat exchanger, or cold box, may also act as a reboiler for the dehydrogenation column. The propylene product leaving the cold box is fed to a deethanizer where C2 and lighter components are separated from the C3 components. The C3 components are then fed to a C3 splitter where propane is separated from propylene. Propylene is recovered as the process product while propane is recycled as propane dehydrogenation feedstock. This process is very energy intensive.

In order to overcome the high energy requirements in both the polymerization system off-gas treatment and the propane dehydrogenation unit, it has been found that combined product and heat integration of a propane dehydrogenation unit with a polypropylene plant may be possible. Embodiments disclosed herein may integrate product flows (propane, propylene, nitrogen) as well as energy streams. It is also proposed, for some embodiments, that process equipment may be shared between the two plants.

Additionally, the energy consumption for the transport of propylene product to the polypropylene plant may be eliminated by close proximity integration. Further, propylene not reacted to form polypropylene, as well as any byproduct propane, may be recycled to the propane dehydrogenation product gas compressor, or to another appropriate location in the propane dehydrogenation plant. Any propane, which is inert in the polypropylene plant reactors, may also be recovered from the polypropylene plant, and returned to the propane dehydrogenation plant with minimal compressor energy required. In one or more embodiments disclosed herein, it has been found that the off-gas from the polypropylene plant may be more effectively utilized, such as by recycle to the dehydrogenation unit without the need for liquefaction or refrigeration, as compared to prior art processes which dispose of such off-gas streams.

Accordingly, disclosed herein is a process integrating a propane dehydrogenation unit with a polypropylene unit, such that the product gas compressor and cold box in the propane dehydrogenation section are shared resources, thus reducing energy consumption of the propane dehydrogenation unit and the polypropylene plant. Overall efficiency of polypropylene production is thereby increased, making the process more economical.

As integrated processes, the polymer-grade propylene product is directly used in the polymerization process to produce polypropylene. In doing so, the propylene transportation or storage costs may be reduced. Additionally, the unreacted propane and propylene in the polypropylene plant may be directly recycled to the propane dehydrogenation unit. Typically with polypropylene plants, the propylene monomer and purge gases need to undergo pressurization and chilling in order to be separated and recycled. In the integrated processes described herein, the need for such energy intensive processes is minimized or negated and the propylene and propane may be directly recycled to the propane dehydrogenation plant, thus minimizing or eliminating compression requirements. As described further below, the process integrates both energy and product flows.

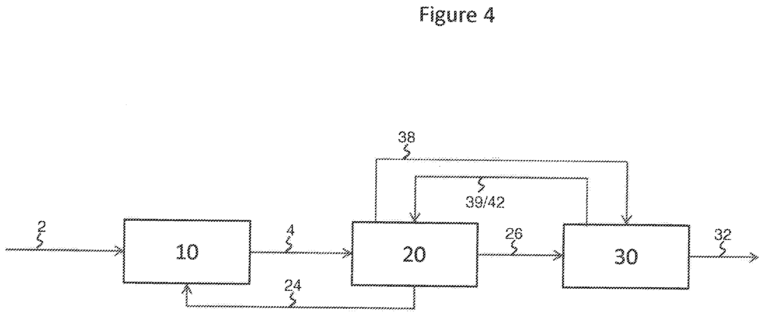

Referring now to Figure 4, a simplified process flow diagram of an integrated process according to embodiments disclosed herein is illustrated. A propane feedstock 2 is fed to a propane dehydrogenation reaction section 10 where a portion of the propane is converted to propylene. The reaction section effluent 4, containing propane and propylene, is fed to a recovery and purification section 20. In the recovery and purification section 20, the propane and propylene are condensed and separated into one or more of a propane recycle stream 24 and a polymer-grade propylene stream 26. This separation may take place in a low pressure separation column operated at a temperature in the range of -5°C to 25°C and a pressure in the range of 4 barg to 10 barg, for example.

The propane recycle stream 24, which may consist essentially of propane, may be recovered from the recovery and purification section 20 and combined with propane feedstock 2 to be used as the feedstock for the propane dehydrogenation reaction section 10. The polymer-grade propylene stream 26, which may have at least 98wt%, at least 99wt%, at least 99.5wt%, or at least 99.8wt% propylene, may be recovered and used as feedstock for polymerization reaction zone 30. A portion of the propylene may also be used in a polymerization reaction zone recycle gas cooler.

In the polymerization reaction zone 30, the polymer-grade propylene stream 26 may be polymerized in the presence of a polymerization catalyst to produce polypropylene. In the polymerization process, propane is an inert component and may also be produced as a byproduct. After polymerization, the polymerized product 32 is fed to a product separation system (not shown).

The product separation system, similar to other separation systems as described above, may include one or more units for separating byproducts and other components, such as propane, unreacted propylene, hydrogen, ethylene, and other components from the product polypropylene. Overall, in the product separation system, the polymerization product is separated into a polypropylene product and one or more purge streams, which may include light gases, monomer, propylene, etc. The monomer and light gases, which may include propane and nitrogen, may be fed back to recovery and purification section 20 via one or more lines 39 and 42. Recovered nitrogen may then be returned to polymerization reaction zone 30 via line 38 for use as a purge gas. Alternatively, propane may be recycled to the dehydrogenation reactor as opposed to the effluent separation system 20.

In some embodiments, the propane in light gas streams 39 and 42 may be at sufficient pressure and quantity to be recycled to the dehydrogenation process with the propane recycle stream 24, or to the dehydrogenation effluent separation system 20, without the need for any intermediate refrigeration or liquefaction. Typically, following propylene polymerization, any propane recovered (i.e., when not flared) needs to be either pressurized or liquefied so the propane can be stored, or sold and shipped to another facility. These processes are extremely energy intensive and increase with the amount of polypropylene being produced. The processes and systems as disclosed herein lack these steps, which may reduce the overall energy consumption of the polymerization process, making the production of polypropylene less capital intensive and more economical.

In prior art polymerization processes, it is often desired to separate unreacted monomer from the nitrogen gas, such that the monomer may be recycled to the polymerization reactor and the nitrogen may be recycled as purge gas, which may be accomplished by a process such as that illustrated in Figure 3, discussed above. This separation process is energy intensive. A condenser is required to chill the gas mixture prior to separation, and a compressor is required to pass the recovered gas through a membrane to separate nitrogen from off-gas. Additionally, the hydrocarbons 304 or a portion thereof may be sent through a distillation column (not illustrated) to avoid buildup of propane and other impurities from the overall system. Further, a compressor is needed for recycling vapor from the reactors and sending it through a cooling water exchanger (recycle gas coolers), which requires a rather complex control system as the gas rate may vary from essentially zero to full design flow.

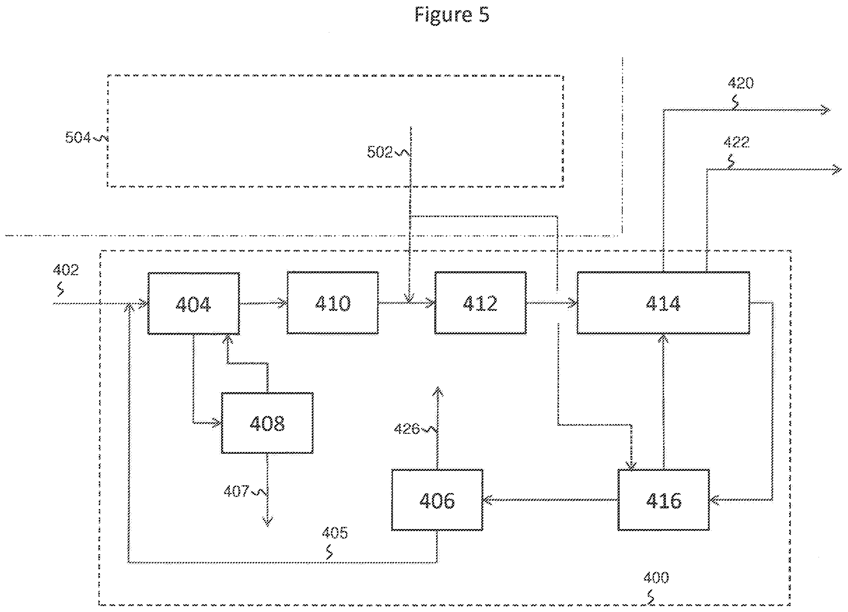

In view of the separation systems proposed for use in propylene polymerization units, integration of the polypropylene off-gas separations with a propane dehydrogenation unit may seem relatively straight-forward. For example, as illustrated in Figure 5, it may be proposed that the carrier gas and/or purge gas steams 502 from the polymerization zone 504 simply be fed to a propane dehydrogenation unit 400 upstream of the product gas compressor 412.

However, this results in a large penalty in compressor size and power requirements. Likewise, one may consider simply feeding the purge gas streams 502 to the deethanizer 416. This, however, results in a large penalty in deethanizer 416 and C3 splitter 406 tower size and utility requirements. As a result, in the past, the traditional approach has been to limit integration of these units on the basis that there is a desirability to operate them independently, and that past integration efforts have led to reduced efficiency, such as for reasons noted above.

In contrast, according to one or more embodiments disclosed herein, the purge gas and carrier gas streams from the polymerization unit may be processed in an efficient, integrated scheme where such penalties are not incurred. Integrating the two systems includes processing the purge gas and carrier gas from the polymerization reaction zone in the propane dehydrogenation zone, and may include supplying refrigerant from the propane dehydrogenation zone to the recycle gas coolers in the polymerization zone. In contrast to prior integration efforts, embodiments herein take advantage of the heretofore unrecognized synergies realizable between the two units.

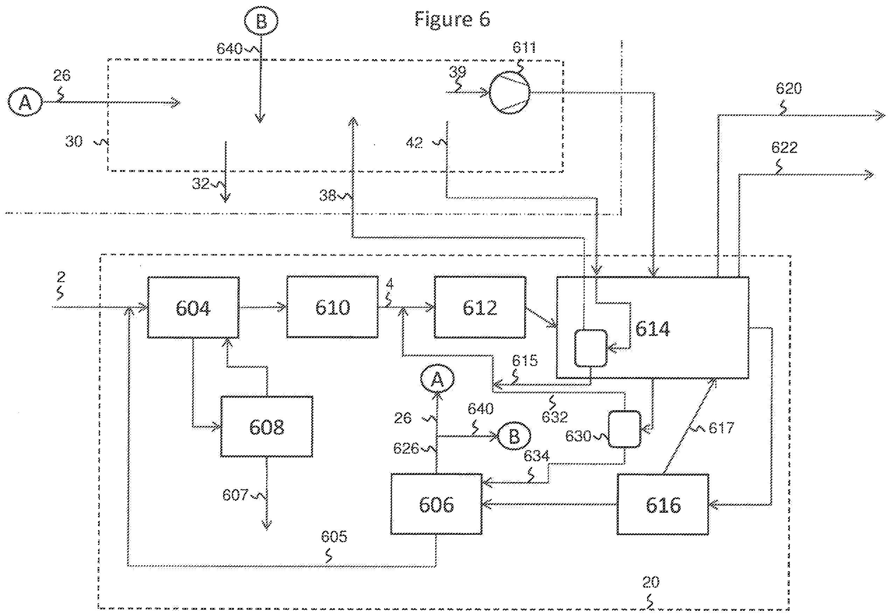

Referring now to Figure 6, a simplified process flow diagram of a process integrating a propane dehydrogenation unit and propylene polymerization unit according to embodiments herein is illustrated. While various portions of the propylene polymerization system and the propane dehydrogenation system are not shown, the manner of integrating the two units is exemplified. One skilled in the art would readily understand, based on the figure and the related description herein those portions not illustrated specifically that may also be modified to integrate the two processes.

A propane dehydrogenation unit 20 may include a vaporizer 604 to vaporize a propane-containing feedstock and recycle propane / heavies 605 recovered from a propane / propylene splitter 606. A deoiler 608 may be used to remove the heavies 607 not vaporized. A portion of the propane is then converted to propylene in reactor 610, the effluent then being compressed in product gas compressor 612, cooled in cold box / exchanger 614, and separated using a deethanizer (C2/C3 splitter) 616 and the C3 splitter 606. Products recovered from propane dehydrogenation unit 20 may include, among others, hydrogen 620, light hydrocarbons 622, and propylene product 626.

The propylene product 626 recovered, or a portion thereof, is then fed via flow line 26 to polypropylene unit 30 and converted to polypropylene or a propylene polymer or copolymer in a polymerization reactor. The polymerization reactor effluent(s) are then separated to result in a polymer product 32, a purge gas 42 and a carrier gas 39. Purge gas 42 comprises nitrogen and, for example, may be a nitrogen-rich gas resulting from polymer drying or headspace composition control for which a relatively larger nitrogen flow is used as compared to the non-nitrogen gas components emanating from the dryers or headspaces. Carrier gas 39 is either leaner in nitrogen relative to stream 42 or does not contain nitrogen at any significant concentration, may include unreacted hydrogen and propylene, and may result from initial separation of the reactor fluidization gases from the polymer product.

Integration of the polymerization unit with the propane dehydrogenation unit according to embodiments herein involves three primary flows, including the purge gas 42, the carrier gas 39, and the propylene product 626.

Purge gas 42 and carrier gas 39 are fed separately to cold box 614. In cold box 614, the purge gas 42 and carrier gas 39 are exchanged against various refrigeration streams, deethanizer 616 overhead stream 617, and the product gas compressor 612 effluent, among other possible streams. As a result of heat exchange in the cold box, hydrocarbons and other components in the purge and carrier gas streams may condense.

Within, as illustrated, or exterior to cold box 614, condensates may be separated from nitrogen in the purge gas. The non-condensed components, which may be essentially only nitrogen, may then be sent back to the polymerization unit 30 via stream 38. Hydrocarbons and other heavies condensed may then be fed to a suitable suction stage of the product gas compressor 612 via flow line 615.

Carrier gas 39 may be compressed in compressor 611 within the polymerization unit prior to heat exchange in cold box 614. Within, or exterior to cold box 614, as illustrated, condensates may be separated from the lighter gases and hydrocarbons in the carrier gas, such as via a flash drum 630. The light hydrocarbons, including ethylene and propylene, and other non-condensed components, including unreacted hydrogen, may then be fed to a suitable suction stage of the product gas compressor 612 via flow line 632, such that the unreacted hydrogen may be efficiently recovered, such as by a pressure swing absorption system (not illustrated), and light hydrocarbons such as ethane and ethylene may be removed by the deethanizer 616.

The integration also re-routes a portion of the propylene product 626 recovered from the C3 splitter 606. A portion of the propylene product 626 is fed to the polymerization reaction zone 30 as high purity propylene feed 26. A portion of the propylene product may also fed via flow line 640 to the polypropylene unit 30 recycle gas coolers, where it may be used as refrigerant (not illustrated). This configuration may eliminate the need for a recycle gas compressor in the recycle gas cooler loop.

With respect to the integration of the purge gas stream, the purge gas from the polymerization unit is compressed and sent to the propane dehydrogenation cold box, where cryogenic separation of the nitrogen and hydrocarbons takes place. The hydrocarbons, mostly propylene, may be fed to the propane dehydrogenation product gas compressor for recovery, while the nitrogen is recycled back to the polymerization unit. As compared to the cold box illustrated in Figure 5, this adds an additional heat exchange pass to the cold box, but it is relatively small compared to the other passes in the cold box (dehydrogenation reactor effluent, deethanizer overheads (which may include a hydrogen-rich stream and a deethanizer overhead product stream), among others). This approach results in the use of the propane dehydrogenation unit to recover a utility stream used in the polymerization unit, with the benefit of eliminating the membrane separation unit as well as reducing compressor requirements.

With respect to the carrier gas, all of the carrier gas from the polymerization unit may be compressed to a relatively low pressure and fed to the propane dehydrogenation cold box, and partially vaporized. The effluent is then passed through a trayed column or a flash drum, depending upon the ethylene content of the carrier gas, to separate light hydrocarbons from C3+ hydrocarbons. The light hydrocarbons are fed to the product gas compressor for recovery, while the C3+ hydrocarbons are sent to the C3 splitter for separation of the propylene. The uniqueness of this approach lies in the processing of the carrier gas in the propane dehydrogenation cold box. This approach results in significantly different product flows and power requirements as compared to the "straightforward" integration of just sending the carrier gas directly to the propane dehydrogenation product gas compressor. In addition to the power benefits, this integration eliminates several pieces of equipment in the polymerization unit, including compressors and distillation equipment.

With respect to the propylene product flows, propylene refrigerant from the propane dehydrogenation unit (propylene product) may be used in the recycle gas coolers of the polymerization unit to condense the recycle gas. Use of the propylene refrigerant allows condensation at a lower pressure and eliminates the need for a recycle gas compressor, which is typically needed to handle non-condensables. One skilled in the art may consider that this would be ineffective, as when the propane dehydrogenation unit is shut down, propylene refrigerant would not be available. However, as long as the propylene refrigerant compressor of the propane dehydrogenation unit is in operation, even if the dehydrogenation reactors are not in service, propylene refrigerant will be available. This integration has the benefit of eliminating the recycle gas compressor in the polymerization unit.

As described above, the cooling and duty requirements to separate the off-gas from the polymerization reaction zone 30 may be met by the recovery and purification section 20. In such embodiments, streams 39 and 42 may be fed to the recovery and purification section 20, where the dehydrogenation unit cold box may serve as the partial condenser for the polymerization off-gas separation system. After being chilled in the cold box, a portion of the resulting streams may be fed to the recovery and purification section 20 separation units with the reactor effluent 4. By feeding the gas streams 39 and 42 in this manner, the deethanizer and C3 splitter in the recovery and purification section 20 may efficiently separate the streams into unreacted monomer, hydrogen, and off-gas. Nitrogen in the off-gas may then be recycled to the polymerization reaction zone 30 as nitrogen feed 38. Nitrogen feed 38 may be used as a purge gas 41. Additionally, a portion of the propylene product may be fed to recycle gas coolers as a refrigerant stream. This refrigerant stream is used to condense a recycle gas, allowing condensation at a lower pressure and elimination the need of a recycle gas compressor.

As described above, the gas streams from the polypropylene zone may be recycled to the propane dehydrogenation zone. This configuration is generally considered counterintuitive and not practiced because of the added capital cost associated with transport piping. Further, this configuration will require both facilities to be located in relatively close proximity. However, the overall energy consumption of the propane dehydrogenation plant and polymerization plant can be significantly reduced, and the capital costs for the polymerization unit may be significantly reduced.

As an example, in Table 1 the operating costs of a standalone propane dehydrogenation unit and a standalone polypropylene unit are compared to the operating costs of an integrated process according to embodiments herein. It is estimated that the difference between integrated and stand-alone utility consumption is about US$0.5 MM per year utility operating cost savings. Additionally, due to the integration and elimination of or changes in size of various pieces of equipment, it is estimated that capital costs may be reduced by over US$15 MM.

**Table 1**

| | | | **Standalone PDH** | | **Standalone PP** | | **Integrated PDH-PP** | |
|---|---|---|---|---|---|---|---|---|
| ***Utility Cost*** | Unit of Utility | Price | Utility Consumption per MT of Propylene Product | Cost per MT of Propylene Product | Utility Consumption per MT of PP Product | Cost per MT of PP Product | Utility Consumption per MT of PP Product | Cost per MT of PP Product |
| **Total Cost** | **US$ / MT** | | | **43** | | **23** | | **65** |
| **Total Cost** | **MMUS$ / year** | | | **19.2** | | **10.5** | | **29.2** |

Advantageously, it has been found that by using product gas compressor and heat exchangers as common equipment between the dehydrogenation reaction zone and the polymerization zone, the capital cost and overall energy requirements are significantly reduced. A typical polypropylene production facility will normally require a separate set of compressors, gas cooling equipment, and fractionation equipment. However, by integrating the polypropylene production with the propane dehydrogenation process, a viable and economical way of sharing compression and heating/cooling requirements can be realized.

Additionally, the propane, which is inert in the polymerization plant reactors, may be recovered from the polymerization plant, and returned to the propane dehydrogenation plant. Because the propane is directly recycled, there is no need for pressurization or liquefaction, which also contributes a large portion of the energy requirements of a typical polymerization plant.

As described above, embodiments herein provide an integrated system for the production of polypropylene. Embodiments herein advantageously integrate both product and utility flows, reducing overall energy requirements of the system.

While the disclosure includes a limited number of embodiments, those skilled in the art, having benefit of this disclosure, will appreciate that other embodiments may be devised which do not depart from the scope of the present invention, which is limited only by the attached claims

## Claims

1. A process for the production of polypropylene comprising:
feeding a hydrocarbon feedstock (402; 2) comprising propane to a propane dehydrogenation reaction zone (610) to convert a portion of the propane to propylene, forming a dehydrogenated effluent (4);
separating the dehydrogenated effluent in a separation system (20), forming a propylene stream (426; 626) and a propane stream (405;605);
feeding at least a portion (26) of the propylene stream (426; 626) to a polymerization zone (30), wherein propylene is reacted to produce a polymerization product (32), and a purge gas (42) and a carrier gas (39) are recovered; and
independently feeding the purge gas (42) and carrier gas (39) to the separation system,
wherein the purge gas (42) comprises a gas containing nitrogen and optionally one or more of propane, propylene, ethylene, or hydrogen, the carrier gas (39) comprises one or more of propane, propylene, ethylene, hydrogen, or nitrogen, and when the carrier gas (39) comprises nitrogen, the carrier gas (39) is leaner in nitrogen relative to the purge gas (42),
wherein the separating comprises:
compressing the dehydrogenated effluent in a product gas compressor (412; 612);
cooling the compressed effluent in a cold box (414; 614); and
separating the cooled, compressed effluent in a deethanizer (416; 616) and a C3 splitter (406; 606); and
wherein the process further comprises:
independently passing the purge gas (42) and carrier gas (39) through the cold box (414; 614), thereby cooling the purge gas (42) and carrier gas (39);
condensing, in the cold box (414; 614), a portion of the carrier gas (39) and a portion of the purge gas (42);
recovering a carrier gas condensate stream (634) and a carrier gas vapor stream (632);
feeding the carrier gas condensate stream (634) to the C3 splitter (406; 606) and feeding the carrier gas vapor stream (632) to the product gas compressor (412; 612);
recovering a purge gas condensate stream (615) and a purge gas vapor stream (38) and vaporizing the purge gas condensate stream in the cold box (414; 614);
feeding the vaporized purge gas condensate stream (615) to the product gas compressor (412; 612) and feeding the purge gas vapor stream (38) to the polymerization zone (30).

2. The process of claim 1, wherein the purge gas vapor stream (38) comprises essentially only nitrogen.

3. The process of claim 1, further comprising feeding another portion (640) of the propylene stream to the polymerization zone (30) for use as a refrigerant.

4. The process of claim 1, wherein propylene is reacted to further produce a recycle gas;
the process further comprising:
feeding a second portion of the propylene stream (426; 626) as a refrigerant to a heat exchanger for cooling the recycle gas.

5. The process of claim 1, further comprising feeding the purge gas vapor stream (38) to a degas unit, contacting the purge gas vapor stream (38) with an effluent from a polymerization reactor or portion thereof to separate a contaminant from polymer.

6. The process of claim 5, wherein the contaminant comprises one or more of hydrogen, ethylene, propane, and propylene.

7. The process of claim 1, further comprising:
mixing the carrier gas vapor stream (632) with the dehydrogenated effluent.

8. A system for the integrated production of propylene and polypropylene comprising:
a propane dehydrogenation reaction zone (400; 10) configured to convert at least a portion of a hydrocarbon feedstock (402; 2) comprising propane to propylene, forming a dehydrogenated effluent (4);
a separation system (20) configured to separate the dehydrogenated effluent and form a propane stream (605) and a propylene stream (426; 626); and
a polymerization zone (30) configured to convert at least a portion (26) of the propylene stream (426; 626) to polypropylene and produce a polymerization product, a carrier gas stream (39), and a purge gas stream (42);
a flow line for feeding the carrier gas stream (39) to the separation system; and
a flow line for feeding the purge gas stream (42) to the separation system,
wherein the purge gas (42) comprises a gas containing nitrogen and optionally one or more of propane, propylene, ethylene, or hydrogen, the carrier gas (39) comprises one or more of propane, propylene, ethylene, hydrogen, or nitrogen, and when the carrier gas (39) comprises nitrogen, the carrier gas (39) is leaner in nitrogen relative to the purge gas (42),
wherein the separation system comprises:
a product gas compressor (412; 612) for compressing the dehydrogenated effluent;
a cold box (414; 614) for cooling the compressed effluent;
a deethanizer (416; 616) configured to separate the cooled, compressed effluent to recover a C2- stream and a C3+ stream; and
a C3 splitter (406; 606) configured to produce an overhead product comprising propylene and a bottoms product comprising propane, and
wherein the cold box (414; 614) comprises:
a pass for cooling the purge gas stream (42);
a separator, within or exterior to the cold box, for receiving the cooled purge gas stream and recovering a purge gas condensate stream and a purge gas vapor stream (38);
a pass for vaporizing the purge gas condensate stream; and
a pass for cooling the carrier gas stream (39), producing a carrier gas condensate stream (634) and a carrier gas vapor stream (632);
wherein the system further comprises:
a line for feeding the vaporized purge gas condensate stream (615) to the product gas compressor (412; 612);
a line for feeding the purge gas vapor stream (38) to the polymerization zone (30);
a line for feeding the carrier gas condensate stream (634) to the C3 splitter (406; 606); and
a line for feeding the carrier gas vapor stream (632) to the product gas compressor (412; 612).

9. The system of claim 8, further comprising:
a polypropylene recycle gas cooler; and
a line for feeding a portion of the propylene stream as a refrigerant to the polymerization recycle gas cooler.

## Patentansprüche

1. Verfahren zur Herstellung von Polypropylen, umfassend:
Zuführen eines Kohlenwasserstoff-Einsatzmaterials (402; 2), das Propan umfasst, in eine Propandehydrierungs-Reaktionszone (610), um einen Teil des Propans in Propylen umzuwandeln, wobei ein dehydrierter Abfluss (4) gebildet wird;
Trennen des dehydrierten Abflusses in einem Trennsystem (20), um einen Propylenstrom (426; 626) und einen Propanstrom (405; 605) zu bilden;
Zuführen wenigstens eines Teils (26) des Propylenstroms (426; 626) zu einer Polymerisationszone (30), in der Propylen umgesetzt wird, um ein Polymerisationsprodukt (32) zu erzeugen, und ein Spülgas (42) und ein Trägergas (39) rückgewonnen werden; und
unabhängiges Zuführen des Spülgases (42) und des Trägergases (39) zu dem Trennsystem,
wobei das Spülgas (42) ein Gas umfasst, das Stickstoff und gegebenenfalls eines oder mehrere von Propan, Propylen, Ethylen oder Wasserstoff enthält, das Trägergas (39) eines oder mehrere von Propan, Propylen, Ethylen, Wasserstoff oder Stickstoff umfasst, und, wenn das Trägergas (39) Stickstoff umfasst, das Trägergas (39) ärmer an Stickstoff als das Spülgas (42) ist,
wobei das Trennen umfasst:
Verdichten des dehydrierten Abflusses in einem Produktgasverdichter (412; 612);
Kühlen des verdichteten Abflusses in einem Kühlkasten (414; 614); und
Trennen des gekühlten, komprimierten Abflusses in einem Entethanisator (416; 616) und einem C3-Splitter (406; 606); und
wobei das Verfahren ferner umfasst:
unabhängig Leiten des Spülgases (42) und des Trägergases (39) durch den Kühlkasten (414; 614), um das Spülgas (42) und das Trägergas (39) zu kühlen;
in dem Kühlkasten (414; 614) Kondensieren eines Teils des Trägergases (39) und eines Teils des Spülgases (42);
Rückgewinnen eines Trägergaskondensatstroms (634) und eines Trägergasdampfstroms (632);
Zuführen des Trägergaskondensatstroms (634) zu dem C3-Splitter (406; 606) und Zuführen des Trägergasdampfstroms (632) zu dem Produktgasverdichter (412; 612);
Rückgewinnen eines Spülgaskondensatstroms (615) und eines Spülgasdampfstroms (38) und Verdampfen des Spülgaskondensatstroms in dem Kühlkasten (414; 614);
Zuführen des verdampften Spülgaskondensatstroms (615) zu dem Produktgasverdichter (412; 612) und Zuführen des Spülgasdampfstroms (38) zu der Polymerisationszone (30).

2. Verfahren nach Anspruch 1, wobei der Spülgasdampfstrom (38) im Wesentlichen nur Stickstoff umfasst.

3. Verfahren nach Anspruch 1, ferner umfassend Zuführen eines weiteren Teils (640) des Propylenstroms zu der Polymerisationszone (30) zur Verwendung als Kältemittel.

4. Verfahren nach Anspruch 1, wobei Propylen umgesetzt wird, um ferner ein Rückführgas zu erzeugen;
wobei das Verfahren ferner umfasst:
Zuführen eines zweiten Teils des Propylenstroms (426; 626) als Kältemittel zu einem Wärmetauscher zum Kühlen des Rückführgases.

5. Verfahren nach Anspruch 1, ferner umfassend Zuführen des Spülgasdampfstroms (38) zu einer Entgasungseinheit, Inkontaktbringen des Spülgasdampfstroms (38) mit einem Abfluss aus einem Polymerisationsreaktor oder einem Teil davon, um einen Verunreinigungsstoff von Polymer zu trennen.

6. Verfahren nach Anspruch 5, wobei der Verunreinigungsstoff eines oder mehrere von Wasserstoff, Ethylen, Propan und Propylen umfasst.

7. Verfahren nach Anspruch 1, ferner umfassend:
Mischen des Trägergasdampfstroms (632) mit dem dehydrierten Abfluss.

8. System zur integrierten Herstellung von Propylen und Polypropylen, umfassend:
eine Propandehydrierungs-Reaktionszone (400; 10), dafür gestaltet, wenigstens einen Teil eines Kohlenwasserstoff-Einsatzmaterials (402; 2), das Propan umfasst, in Propylen umzuwandeln, wobei ein dehydrierter Abfluss (4) gebildet wird;
ein Trennsystem (20), dafür gestaltet, den dehydrierten Abfluss zu trennen und einen Propanstrom (605) und einen Propylenstrom (426; 626) zu bilden; und
eine Polymerisationszone (30), dafür gestaltet, wenigstens einen Teil (26) des Propylenstroms (426; 626) in Polypropylen umzuwandeln und ein Polymerisationsprodukt, einen Trägergasstrom (39) und einen Spülgasstrom (42) zu erzeugen;
eine Strömungsleitung zum Zuführen des Trägergasstroms (39) zu dem Trennsystem; und
eine Strömungsleitung zum Zuführen des Spülgasstroms (42) zu dem Trennsystem,
wobei das Spülgas (42) ein Gas umfasst, das Stickstoff und gegebenenfalls eines oder mehrere von Propan, Propylen, Ethylen oder Wasserstoff enthält, das Trägergas (39) eines oder mehrere von Propan, Propylen, Ethylen, Wasserstoff oder Stickstoff umfasst, und, wenn das Trägergas (39) Stickstoff umfasst, das Trägergas (39) ärmer an Stickstoff als das Spülgas (42) ist,
wobei das Trennsystem umfasst:
einen Produktgasverdichter (412; 612) zum Verdichten des dehydrierten Abflusses;
einen Kühlkasten (414; 614) zum Kühlen des komprimierten Abflusses;
einen Entethanisator (416; 616), dafür gestaltet, den gekühlten, komprimierten Abfluss zu trennen, um einen C2--Strom und einen C3+-Strom zu gewinnen; und
einen C3-Splitter (406; 606), dafür gestaltet, ein Kopfprodukt, das Propylen umfasst, und ein Sumpfprodukt, das Propan umfasst, herzustellen, und
wobei der Kühlkasten (414; 614) umfasst:
einen Durchgang zum Kühlen des Spülgasstroms (42);
einen Abscheider innerhalb oder außerhalb des Kühlkastens zum Aufnehmen des gekühlten Spülgasstroms und Gewinnen eines Spülgaskondensatstroms und eines Spülgasdampfstroms (38);
einen Durchgang zum Verdampfen des Spülgaskondensatstroms; und
einen Durchgang zum Kühlen des Trägergasstroms (39), um einen Trägergaskondensatstrom (634) und ein Trägergasdampfstrom (632) zu erzeugen;
wobei das System ferner umfasst:
eine Leitung zum Zuführen des verdampften Spülgaskondensatstroms (615) zu dem Produktgasverdichter (412; 612);
eine Leitung zum Zuführen des Spülgasdampfstroms (38) zu der Polymerisationszone (30);
eine Leitung zum Zuführen des Trägergaskondensatstroms (634) zu dem C3-Splitter (406; 606); und
eine Leitung zum Zuführen des Trägergasdampfstroms (632) zu dem Produktgasverdichter (412; 612).

9. System nach Anspruch 8, ferner umfassend:
einen Polypropylen-Rückführgaskühler; und
eine Leitung zum Zuführen eines Teils des Propylenstroms als Kältemittel zu dem Polymerisations-Rückführgaskühler.

## Revendications

1. Procédé pour la production de polypropylène comprenant :
l'alimentation d'une charge hydrocarbonée (402 ; 2) comprenant du propane vers une zone de réaction de déshydrogénation du propane (610) afin de convertir une partie du propane en propylène, formant un effluent déshydrogéné (4) ;
la séparation de l'effluent déshydrogéné dans un système de séparation (20), formant un courant de propylène (426 ; 626) et un courant de propane (405 ; 605) ;
l'alimentation d'au moins une partie (26) du flux de propylène (426 ; 626) vers une zone de polymérisation (30), dans laquelle le propylène est mis en réaction pour produire un produit de polymérisation (32), et un gaz de purge (42) et un gaz vecteur (39) sont récupérés ; et
l'alimentation indépendante du gaz de purge (42) et du gaz vecteur (39) vers le système de séparation,
dans lequel le gaz de purge (42) comprend un gaz contenant de l'azote et, optionnellement, un ou plusieurs des éléments suivants : propane, propylène, éthylène ou hydrogène, le gaz vecteur (39) comprend un ou plusieurs des éléments suivants : propane, propylène, éthylène, hydrogène ou azote, et lorsque le gaz vecteur (39) comprend de l'azote, le gaz vecteur (39) est moins riche en azote que le gaz de purge (42),
dans lequel la séparation comprend :
la compression de l'effluent déshydrogéné dans un compresseur de gaz de produit (412 ; 612) ;
le refroidissement de l'effluent comprimé dans un caisson frigorifique (414 ; 614) ; et
la séparation de l'effluent refroidi et comprimé dans un déséthaniseur (416 ; 616) et un séparateur C3 (406 ; 606) ; et
dans lequel le procédé comprend en outre :
le passage indépendant du gaz de purge (42) et du gaz vecteur (39) à travers le caisson frigorifique (414 ; 614), refroidissant ainsi le gaz de purge (42) et le gaz vecteur (39) ;
la condensation, dans le caisson frigorifique (414 ; 614), d'une partie du gaz vecteur (39) et d'une partie du gaz de purge (42) ;
la récupération d'un flux de condensat du gaz vecteur (634) et d'un flux de vapeur du gaz vecteur (632) ;
l'alimentation du flux de condensat du gaz vecteur (634) vers le séparateur C3 (406; 606) et l'alimentation du flux de vapeur du gaz vecteur (632) vers le compresseur de gaz produit (412; 612);
la récupération d'un courant de condensat du gaz de purge (615) et d'un courant de vapeur du gaz de purge (38) ; et
la vaporisation du flux de condensat du gaz de purge dans le caisson frigorifique (414 ; 614) ;
l'alimentation du flux de condensat vaporisé du gaz de purge (615) vers le compresseur du gaz produit (412 ; 612) et alimentation du flux de vapeur du gaz de purge (38) vers la zone de polymérisation (30).

2. Le procédé selon la revendication 1, dans lequel le flux de vapeur du gaz de purge (38) comprend essentiellement uniquement de l'azote.

3. Le procédé de la revendication 1, comprenant en outre l'alimentation d'une autre portion (640) du flux de propylène vers la zone de polymérisation (30) en vue de son utilisation comme réfrigérant.

4. Le procédé de la revendication 1, dans lequel le propylène est mis en réaction pour produire en outre un gaz de recyclage ;
le procédé comprenant en outre :
l'alimentation d'une seconde portion du flux de propylène (426 ; 626) en tant que réfrigérant vers un échangeur de chaleur pour refroidir le gaz de recyclage.

5. Le procédé selon la revendication 1, comprenant en outre l'alimentation du flux de vapeur du gaz de purge (38) vers une unité de dégazage, la mise en contact du flux de vapeur du gaz de purge (38) avec un effluent d'un réacteur de polymérisation ou d'une partie de celui-ci afin de séparer un contaminant du polymère.

6. Le procédé de la revendication 5, dans lequel le contaminant comprend un ou plusieurs des éléments suivants : l'hydrogène, l'éthylène, le propane et le propylène.

7. Procédé selon la revendication 1, comprenant en outre :
le mélange du flux de vapeur de gaz vecteur (632) avec l'effluent déshydrogéné.

8. Un système pour la production intégrée de propylène et de polypropylène comprenant :
une zone de réaction de déshydrogénation du propane (400 ; 10) configurée pour convertir au moins une partie d'une charge hydrocarbonée (402 ; 2) comprenant du propane en propylène, formant un effluent déshydrogéné (4) ;
un système de séparation (20) configuré pour séparer l'effluent déshydrogéné et former un flux de propane (605) et un flux de propylène (426 ; 626) ; et
une zone de polymérisation (30) configurée pour convertir au moins une portion (26) du flux de propylène (426 ; 626) en polypropylène et produire un produit de polymérisation, un courant de gaz vecteur (39), et un courant de gaz de purge (42) ;
une conduite d'alimentation pour acheminer le flux de gaz vecteur (39) vers le système de séparation ; et
une canalisation d'alimentation pour acheminer le flux de gaz de purge (42) vers le système de séparation,
dans lequel le gaz de purge (42) comprend un gaz contenant de l'azote et optionnellement un ou plusieurs de propane, propylène, éthylène ou hydrogène, le gaz vecteur (39) comprend un ou plusieurs de propane, propylène, éthylène, hydrogène ou azote, et lorsque le gaz vecteur (39) comprend de l'azote, le gaz vecteur (39) est plus pauvre en azote par rapport au gaz de purge (42),
dans lequel le système de séparation comprend :
un compresseur de gaz produit (412 ; 612) pour comprimer l'effluent déshydrogéné ;
un caisson frigorifique (414 ; 614) pour refroidir l'effluent comprimé ;
un déséthaniseur (416 ; 616) configuré pour séparer l'effluent refroidi, comprimé afin de récupérer un courant C2- et un courant C3+ ; et
un séparateur C3 (406 ; 606) configuré pour produire un produit en tête comprenant du propylène et un produit de fond comprenant du propane, et
dans lequel le caisson frigorifique (414 ; 614) comprend :
une passe pour refroidir le flux de gaz de purge (42) ;
un séparateur, à l'intérieur ou à l'extérieur du caisson frigorifique, pour recevoir le flux de gaz de purge refroidi et récupérer un flux de condensat du gaz de purge et un flux de vapeur du gaz de purge (38) ;
une passe pour vaporiser le flux de condensat du gaz de purge ; et
une passe pour refroidir le flux de gaz vecteur (39), produisant un flux de condensat du gaz vecteur (634) et un flux de vapeur du gaz vecteur (632) ;
dans lequel le système comprend en outre :
une conduite pour alimenter le flux de condensat vaporisé du gaz de purge (615) vers le compresseur de gaz produit (412 ; 612) ;
une conduite d'alimentation du flux de vapeur du gaz de purge (38) vers la zone de polymérisation (30) ;
une conduite d'alimentation du flux de condensat du gaz vecteur (634) vers le séparateur C3 (406 ; 606) ; et
une conduite pour alimenter le flux de vapeur du gaz vecteur (632) vers le compresseur de gaz produit (412 ; 612).

9. Système selon la revendication 8, comprenant, en outre :
un refroidisseur de gaz de recyclage de polypropylène ; et
une conduite pour alimenter une portion du flux de propylène en tant que réfrigérant vers le refroidisseur du gaz de recyclage de polymérisation.
